# EUROPEAN PATENT SPECIFICATION

(11) **EP 2 185 158 B1**
(45) Date of publication and mention of the grant of the patent: **25.12.2013**
(21) Application number: 08785257.0
(22) Date of filing: 31.07.2008
(51) Int. Cl.: A61K 31/685, A61P 1/00

(54) **USE OF PHOSPHATIDYLCHOLINE FOR TREATING STEROID-DEPENDENT AND STEROID-REFRACTORY ULCERATIVE COLITIS**
VERWENDUNG VON PHOSPHATIDYLCHOLIN ZUR BEHANDLUNG VON STEROIDABHÄNGIGER UND STEROID-REFKRAKTÄRER COLITIS ULCEROSA
UTILISATION DE PHOSPHOTIDYLCHOLINE POUR LE TRAITEMENT DE LA COLITE ULCÉREUSE DÉPENDANTE DES STÉROÏDES OU RÉFRACTAIRE AUX STÉROÏDES

(30) Priority: 31.07.2007 US 953146 P
(43) Date of publication of application: 19.05.2010
(73) Proprietor: PAT GmbH, 81375 München (DE)
(72) Inventor:
(74) Representative: Jacobi, Markus Alexander
(86) International application number: PCT/EP2008/006315
(87) International publication number: WO 2009/015891

(56) References cited:
- US-B1- 6 677 319
- STREMMEL W ET AL: "Retarded release phosphatidylcholine benefits patients with chronic active ulcerative colitis" GUT, BRITISH MEDICAL ASSOCIATION, LONDON, vol. 54, no. 7, 1 July 2005 (2005-07-01), pages 966-971, XP002414502 ISSN: 0017-5749 cited in the application
- STURM A ET AL: "Modulation of gastrointestinal wound repair and inflammation by phospholipids" BIOCHIMICA AND BIOPHYSICA ACTA. MOLECULAR AND CELL BIOLOGY OFLIPIDS, ELSEVIER, AMSTERDAM, NL, vol. 1582, no. 1-3, 23 May 2002 (2002-05-23), pages 282-288, XP004366551 ISSN: 1388-1981
- STREMMEL WOLFGANG ET AL: "Phosphatidylcholine for steroid-refractory chronic ulcerative colitis: a randomized trial." ANNALS OF INTERNAL MEDICINE 6 NOV 2007, vol. 147, no. 9, 6 November 2007 (2007-11-06), pages 603-610, XP002506765 ISSN: 1539-3704

## Description

### FIELD OF THE INVENTION

In general, the present invention relates to the fields of medicine and pharmacy. More particularly, the present invention relates to phosphatidylcholines for use in methods for treating individuals having steroid-dependent or steroid refractory ulcerative colitis, using medications containing phosphatidylcholine.

### BACKGROUND

Chronic inflammatory bowel diseases, such as ulcerative colitis, affect in high degree young and medium-age adults with increasing frequency. Approximately 1 in 100 adults aged 20-40 are affected by ulcerative colitis. The disease is primarily characterized by frequent bouts of watery diarrhea often accompanied by blood and/or mucus. Other symptoms include cramping, abdominal pain, pain on opening the bowels, urgent and frequent need to open the bowels, the sensation of incomplete emptying of the bowels, nausea, loss of appetite, weight loss, extreme tiredness, skin rashes, mouth ulcers, joint pains and anemia. When the disease affects only the rectum, it is known as proctitis. When ulcerative colitis affects more of the colon than the rectum alone, symptoms are typically more severe. In most cases, the symptoms vary according to the degree of inflammation in the bowel and whether or not the lining of the bowel has become ulcerated. Chronic ulcerative colitis can cause changes in the liver (sclerosing cholangitis) and increase the risk of developing bowel cancer.

The cause of ulcerative colitis is not fully understood, however, there does appear to be a genetic component as the disease tends to run in families and 10 to 20% of people with ulcerative colitis are likely to have at least one other person in their family affected. Treatment of ulcerative colitis is focused on controlling flare-ups as quickly as possible and reducing the chances of further flare-ups or complications. Mesalamine (5-aminosalicylic acid; 5-ASA) is a standard first-line therapy for mild-to-moderate ulcerative colitis. The main treatments for ulcerative colitis flare-ups include steroid therapy, the use of immunosuppressant, and in the most extreme cases, surgery. Although steroids are often effective at reducing bouts of inflammation, adverse side effects are associated with long term steroid use. In spite of the adverse side effects, some individuals have such a severe progression of the disease that long term steroid use is required. Such individuals are characterized as having steroid-dependent ulcerative colitis. Other individuals do not favorably respond or become substantially irresponsive to steroid treatment, and thus, they are said to have a steroid-refractory disease. For individuals having a steroid-dependent or steroid-refractory disease, removal of the colon may be necessary. As such, it would be beneficial to provide a treatment for steroid-dependent and/or steroid-refractory ulcerative colitis that has improved efficacy or less severe consequences than the limited treatment options available to individuals having these conditions.

### SUMMARY

One embodiment of the present invention relates to phosphatidylcholines for use in a method of ameliorating at least one symptom of ulcerative colitis. The method comprises a first step of identifying an individual having steroid-dependent ulcerative colitis and a second step of administering to the individual a therapeutically effective amount of phosphatidylcholine, thereby ameliorating at least one symptom of the ulcerative colitis.

Another embodiment of the present invention relates to phosphatidylcholines for use in a method of ameliorating at least one symptom of ulcerative colitis, which comprises a first step of identifying an individual having steroid-dependent ulcerative colitis that has become steroid-refractory ulcerative colitis and a second step of administering to the individual a therapeutically effective amount of phosphatidylcholine, thereby ameliorating at least one symptom of the ulcerative colitis.

In another embodiment, the present invention relates to phosphatidylcholines for use in a method of ameliorating at least one symptom of ulcerative colitis comprising the steps of identifying an individual having steroid-refractory ulcerative colitis and then administering to the individual a therapeutically effective amount of phosphatidylcholine, thereby ameliorating at least one symptom of the ulcerative colitis.

With respect to each of the above embodiments, the phosphatidylcholine can be administered in a delayed release oral formulation. In a preferred embodiment, the delayed release is pH-dependent.

The pH-dependent delayed release formulations used in the above-described methods can comprise one or more gastric acid resistant substances. For example, the gastric acid resistant substances can include film covershields and carrier matrices. In a preferred embodiment, the gastric acid resistant substance comprises an acrylpolymer, such as Eudragit. In such embodiments Eudragit L and/or Eudragit S are particularly preferred. An especially preferred acrylpolymer is Eudragit S100. Another especially preferred acrylpolymer is Eudragit L30. Exemplary preferred pH-dependent delayed release formulations comprise 500 mg phosphatidylcholine coated with Eudragit S100, Eudragit L30 or a mixture of the two polymers.

In embodiments of the above-described methods, the therapeutically effective amount of phosphatidylcholine that is administered to the individual ranges from about 100 mg to about 8 g per day. A preferred range includes from about 500 mg to about 4 g per day. In an especially preferred embodiment, the therapeutically effective amount of phosphatidylcholine that is administered to the individual is about 2 g per day. In some embodiments, the phosphatidylcholine is administered near meal time. The frequency of administration of the phosphatidylcholine can be from about 1 to about 8 times per day with a preferred administration frequency being about 4 times per day or less. Administration may be continued for at least 1 week, at least 2 weeks, or as long as needed throughout the life of the individual.

In some embodiments of the present invention, administration of phosphatidylcholine as set forth in the above-described methods can result in a quality of life index score improvement of at least 25%, a histology index reduction of at least 25%, an endoscopic activity index improvement of at least 50%, a clinical activity index improvement of at least 50%, or in some cases, cessation of clinically active ulcerative colitis.

In certain methods described herein where the individual suffers from steroid-dependent ulcerative colitis, the steroid-dependence can be ameliorated by at least about a 30% dose reduction of steroids administered to the individual as treatment for ulcerative colitis. In other embodiments, the reduction in steroids dose can be at least about 50%. In preferred embodiments, the dose reduction can be at least about 75%, and in especially preferred embodiments, as much as 100%.

Other embodiments of the present invention relate to for use phosphatidylcholine in the preparation of a medicament for ameliorating at least one symptom of ulcerative colitis in an individual identified as having steroid-dependent ulcerative colitis.

Further embodiments relate to phosphatidylcholine for use in the preparation of a medicament for ameliorating at least one symptom of ulcerative colitis in an individual identified as having steroid-dependent ulcerative colitis that has become steroid-refractory ulcerative colitis.

Still other embodiments of the present invention relate to phosphatidylcholine for use in the preparation of a medicament for ameliorating at least one symptom of ulcerative colitis in an individual identified as having steroid-refractory ulcerative colitis.

In certain embodiments of each of the uses described above, the phosphatidylcholine may be formulated as a delayed release medicament for oral administration. In a preferred embodiment, the delayed release is pH-dependent.

In some embodiments of the above-described uses the pH-dependent delayed release medicaments can comprise one or more gastric acid resistant substance. For example, the gastric acid resistant substances can include film covershields and carrier matrices. In a preferred embodiment, the gastric acid resistant substance comprises an acrylpolymer, such as Eudragit. In such embodiments Eudragit L and/or Eudragit S are particularly preferred. An especially preferred acrylpolymer is Eudragit S100. Another especially preferred acrylpolymer is Eudragit L30. Exemplary preferred pH-dependent delayed release medicaments comprise 500 mg phosphatidylcholine coated with Eudragit S100, Eudragit L30 or a mixture of the two polymers.

### BRIEF DESCRIPTION OF THE DRAWINGS

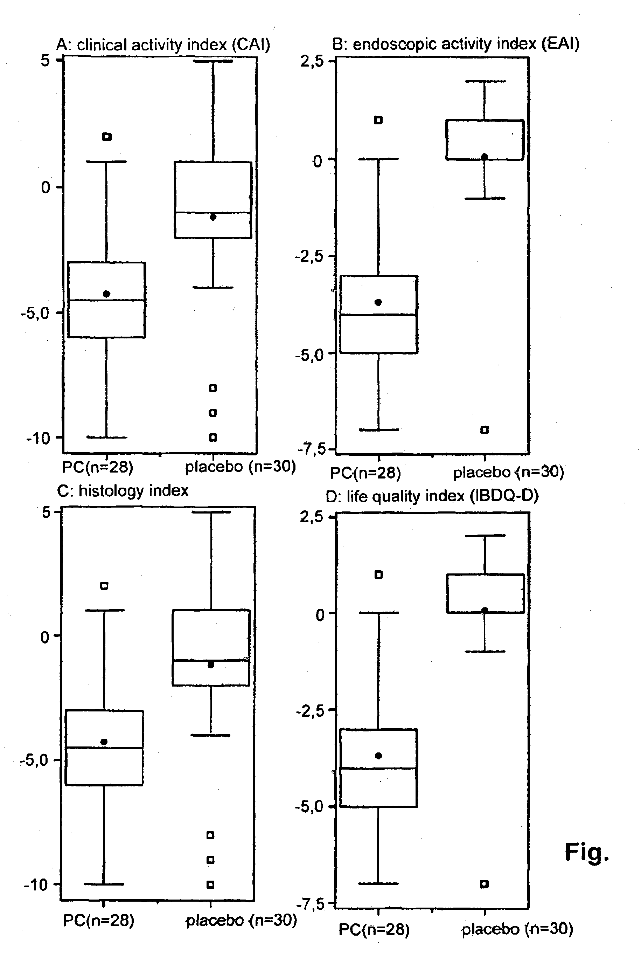

Figures 1A-D are box-and-whisker plots showing the reduction in secondary endpoint variables in patient populations treated with phosphatidylcholine as compared to patient populations treated with placebo. Panel A - reduction in clinical activity index (CAI); Panel B - reduction in endoscopic activity index (EAI); Panel C - reduction in histology index (HI); and Panel D - reduction in life quality index (LQD or IBDQ-D).

### DETAILED DESCRIPTION

Corticosteroids have been used to treat ulcerative colitis for decades and remain the most effective treatment for controlling an acute disease flare-up. Despite the efficacy of steroids for most individuals having ulcerative colitis, they are not a panacea. Some individuals may demonstrate steroid-dependence (clinical relapse upon withdrawal of steroids), whilst others may exhibit a disease that is steroidresistant or steroid-refractory (inadequate or no response to steroid treatment). Individuals having steroid-dependent ulcerative colitis must cope with the undesirable side effects of long term corticosteroid use, which include, among other things, impaired glucose tolerance, osteoporosis, hypertension and skin changes. Furthermore, some individuals that fail to adequately respond to steroids will still suffer from steroid-related side effects. This is often a contributing factor in the decision to add further immunosuppressive treatments, or to proceed to surgery.

Steroids are powerful, potentially toxic drugs that reduce inflammation and suppress the body's immune system. Although a number of different steroids can be used, prednisone and prednisolone are the most commonly prescribed steroids for treatment of ulcerative colitis. While they are very helpful in the management of the disease, steroids can produce a number of side effects ranging from annoying to dangerous. Annoying side effects include puffiness in the face, acne, insomnia, tremors, night sweats, weight gain, and mood disturbances. Dangerous side effects include increased blood pressure, osteoporosis, severe depression, and even psychosis. Long-term steroid use can lead to cataracts and glaucoma. Steroids are typically used to treat moderate-to-severe symptoms during a flare-up and individuals are weaned off of them as quickly as possible after remission is achieved. Unfortunately, some individuals never achieve remission.

Once the decision is made to use oral corticosteroids, treatment usually is initiated with prednisone or an equivalent steroid at a dose of 40-60 mg daily. The majority of individuals with ulcerative colitis respond with an improvement in symptoms. Once symptoms improve, prednisone or its equivalent is usually reduced by 5-10 mg per week until the dose of 20 mg per day is reached. The dose then is tapered at a slower rate until the prednisone ultimately is discontinued. Although this is a typical dose regimen, the standard typical dose varies by severity of symptoms and response to drug therapy.

Applicants have surprisingly found that administration of phosphatidylcholine to individuals identified as having chronic ulcerative colitis that is steroid-dependent can serve as a replacement for steroids in a therapeutic regimen for the treatment of ulcerative colitis. The fact that phosphatidylcholine can replace steroids in steroid-dependent ulcerative colitis creates the possibility of a powerful and relatively safe therapy that avoids the severe side effects of long-term steroid administration in individuals suffering from ulcerative colitis. Also surprising was the discovery that administration of phosphatidylcholine to steroid-refractory chronic active ulcerative colitis individuals ameliorated symptoms of ulcerative colitis. This discovery creates the possibility of an alternative drug to combat ulcerative colitis in individuals that, until now, had very limited therapeutic options.

By steroid-dependent ulcerative colitis is meant repeated episodes of clinical relapse upon withdrawal of steroid treatment after a standard course of steroid treatment. Alternatively, by steroid-dependent ulcerative colitis is meant a chronic condition that requires steroid therapy for time periods substantially longer than those required for the treatment of intermittent acute flare-ups. Individuals suffering from steroid-dependent ulcerative colitis must continue the substantial use of steroids or face relapse of the symptoms associated with ulcerative colitis. Some embodiments of the present invention thus are useful in methods of ameliorating at least one symptom of steroid-dependent ulcerative colitis in an individual suffering from steroid-dependent ulcerative colitis. In particular, such embodiments are useful in methods of ameliorating at least one symptom of ulcerative colitis by first identifying an individual having steroid-dependent ulcerative colitis and then administering to the individual a therapeutically effective amount of phosphatidylcholine so as to ameliorate at least one symptom of the ulcerative colitis. In some embodiments, administration of phosphatidylcholine to an individual having steroid dependent ulcerative colitis ameliorates the steroid-dependence by a steroid dose reduction of at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or up to 100%. Accordingly, in some embodiments complete steroid withdrawal is achieved with treatment of steroid-dependent ulcerative colitis with phosphatidylcholine.

With respect to individuals not having the steroid-dependent disease, steroid-refractory ulcerative colitis refers to ulcerative colitis with symptoms that fail to respond to steroid treatment within about 7 to about 14 days. In the case of individuals having ulcerative colitis that is characterized as steroid-dependent, steroid-refractory ulcerative colitis means that the disease no longer adequately responds to steroid therapy. Individuals suffering from steroid-dependent ulcerative colitis that becomes steroid-refractory can no longer turn to steroids to adequately ameliorate symptoms associated with the disease. Embodiments of the present invention thus are useful in methods of ameliorating of at least one symptom of ulcerative colitis in an individual suffering from steroid-refractory ulcerative colitis and methods of ameliorating at least one symptom of steroid-refractory ulcerative colitis in an individual having steroid-dependent ulcerative colitis that has become steroid-refractory ulcerative colitis. In one particular embodiment, the present invention is useful in methods of ameliorating at least one symptom of ulcerative colitis by identifying an individual having steroid-refractory ulcerative colitis and then administering to the individual a therapeutically effective amount of phosphatidylcholine so as to ameliorate at least one symptom of the ulcerative colitis. In another particular embodiment, methods of ameliorating at least one symptom of ulcerative colitis by first identifying an individual having steroid-dependent ulcerative colitis that has become steroid-refractory ulcerative colitis and then administering to the individual a therapeutically effective amount of phosphatidylcholine so as to ameliorate at least one symptom of the ulcerative colitis are contemplated.

Each of the methods described herein rely on the administration of phosphatidylcholine (PC) to achieve their therapeutic benefit. Phosphatidylcholine (PC) is a phospholipid having the following schematic structural formula: 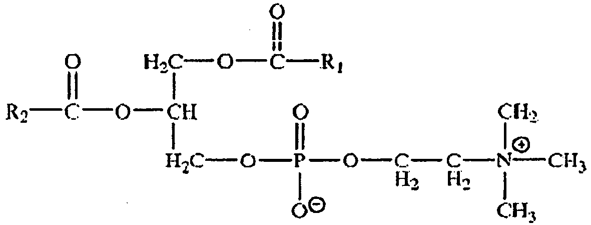 Where R, is a saturated or unsaturated fatty acid with 12-24 carbon atoms, and R₂ is a saturated or unsaturated fatty acid with 12-24 carbon atoms.

One convenient source of phosphatidylcholine is lecithin. In some embodiments of the present invention, the term "lecithin" is used to refer specifically to phosphatidylcholine. In other embodiments, lecithin refers to a mixture of lipids containing phosphatidylcholine. Lecithin can be found in, for example, soy, eggs, dairy products, and meats. In each of the embodiments of the methods described herein, a therapeutically effective amount of phosphatidylcholine is administered to the identified steroid-refractory or steroid-dependent individuals in order to achieve the therapeutic benefit. In some embodiments of the present invention, a therapeutically effective amount ranges from about 100 mg to about 8 g phosphatidylcholine per day. Doses higher than 8 g per day can be of advantage in certain cases. In other embodiments, the therapeutically effective amount is at least about 100 mg, at least about 250 mg, at least about 500 mg, at least about 750 mg, at least about 1 g, at least about 2 g, at least about 4 g, at least about 6 g or at least about 8 g phosphatidylcholine per day.

In each of the methods described herein, the route of administration of phosphatidylcholine is typically oral or rectal. In some embodiments of rectal administration, the phosphatidylcholine is supplied as a clysma, suppository or foam. Oral applications can be provided in form of suitable formulations, for example, as tablets, capsules, or granules contained in sachets. In some embodiments, the oral administration is a delayed release administration in which the release of phosphatidylcholine begins after the formulation has passed the phospholipase-containing pancreatic juices that enter the descending duodenum through the pancreatic duct to ensure that intact phosphatidylcholine enters the distal parts of the intestine and the colon. In another embodiment, the phosphatidylcholine is released from the oral formulation after its transit through approximately the first two thirds of the small intestine. In such administration, the phosphatidylcholine typically begins to be released in the lower part of the ileum.

In especially preferred embodiments of the present invention, oral formulations of phosphatidylcholine for use in the methods described herein are formulations that produce a pH-dependent delayed release. By using such pH-dependent delayed release oral formulations, the application of phosphatidylcholine occurs in lower ileum sections and colon. Oral formulations that produce such pH-dependent delayed release can be conveniently manufactured by coating phosphatidylcholine cores with covershields and/or carrier matrices which are gastric acid resistant and release the phosphatidylcholine in pH-dependent fashion into the lower ileum or colon. In preferred embodiments, film covershields containing one or more acrylpolymers for pH-dependent delayed release, such as Eudragit, are used. In particularly preferred embodiments, Eudragit L and/or Eudragit S are used to coat the phosphatidylcholine. In some of these preferred embodiments, the coating comprises Eudragit S100. In others, the coating comprises Eudragit L30 and/or Eudragit L30D.

Oral pH-dependent delayed release formulations for use with the methods described herein can be manufactured as tablets, granulates, capsules, pellets or pellet tablets. Such formulations can further contain the usual pharmaceutical excipients including, but not limited to, binders, diluents, carrier substances, flow agents and disintegrants. In such embodiments, the phosphatidylcholine is present in each unit dose of the formulation in an amount ranging from about 25 mg to about 2 g. In a preferred embodiment, the unit dose of the formulation comprises about 500 mg phosphatidylcholine.

In a preferred embodiment of the present invention, sachets or other containers holding pH-dependent delayed release granules comprising phosphatidylcholine coated with Eudragit are provided. The granules are then administered to individuals according to the methods described herein. In another preferred embodiment, the phosphatidylcholine is packed in a high volume in gelatin capsules. The gelatin capsules are then coated with Eudragit or another acrylpolymer to promote pH-dependent delayed release. In yet another preferred embodiment, granules or pellets of phosphatidylcholine are coated with Eudragit or another acrylpolymer to promote pH-dependent delayed release. These granules or pellets are then packed in coated or uncoated gelatin capsules. Eudragit S and L-formulations (e.g. Eudragit S100, Eudragit L100, Eudragit L30, and/or Eudragit L30D), alone or in combination, may be used to promote a delayed release at pH ≥ 6.4, as is present in terminal ileum. The use of Eudragit formulations and their mixtures (L-, S- and R-formulations) for pH-dependent delayed release is known in the art. For example, embodiments describing pH-dependent delayed release phosphatidylcholine formulations are described in U.S. Patent No. 6,677,319.

In some of the methods described herein, phosphatidylcholine is administered to an individual in an amount ranging from about 100 mg to about 8 g per day, although higher doses can be of advantage in certain cases. In a preferred embodiment, the amount administered ranges from about 500 mg to about 4 g per day. In an even more preferred embodiment, the amount of phosphatidylcholine that is administered ranges from about 1 g to about 2 g per day. In an especially preferred embodiment, the amount of phosphatidylcholine that is administered to an individual is about 2 g per day.

In the methods described herein, the phosphatidylcholine formulations can be administered from once to several times daily. In one embodiment, the formulations are administered from about 1 to about 8 times per day, preferably the formulations are administered from about 2 to about 6 times per day, more preferably the formulations are administered from about 3 to about 5 times per day, even more preferably the formulations are administered about 4 times per day.

In accordance with the methods described herein, phosphatidylcholine formulations can be orally administered with or without food. In a preferred embodiment, an oral pH-dependent delayed release formulation is administered near meal time. In one embodiment, the formulation can be administered about 3 hours before a meal, preferably about 2 hours before a meal, more preferably about 1 hour before a meal, and even more preferably immediately before the meal. In another embodiment, the formulation is administered during a meal. In yet another embodiment, the formulation is administered after a meal. In such embodiments, the formulation can be administered about 3 hours after a meal, preferably about 2 hours after a meal, more preferably about 1 hour after a meal, and even more preferably immediately after the meal.

A preferred method for carrying out the above-described administration is to provided the individual with about 500 mg of phosphatidylcholine about 4 times per day. However, it will be appreciated that the amount of phosphatidylcholine provided in each dose, the number of doses per day and the total daily amount of phosphatidylcholine administered may vary based on the severity of symptoms and degree of amelioration of symptoms in response to dosing regimen.

In some embodiments of the present invention, the duration of treatment varies based on severity of symptoms progression of the disease in response to the treatment. In accordance with each of the methods described herein, administration of phosphatidylcholine can be continued for at least about 1 week, at least about 2 weeks, at least about 3 weeks, at least about 4 weeks, at least about 1 month, at least about 2 months, at least about 3 months, at least about 4 months, at least about 5 months, at least about 6 months, at least about 7 months, at least about 8 months, at least about 9 months, at least about 10 months, at least about 11 months, or at least about 12 months. In other embodiments, administration of phosphatidylcholine continued as necessary for the life of the individual.

The efficacy of the methods described herein can be assessed by monitoring treated individuals using indices for the assessment of the severity of ulcerative colitis that are known in the art. These indices include, but are not limited to, the clinical activity index (CAI), the endoscopic activity index (EAI), the histology index (HI), the Short Clinical Colitis Activity Index (SCCAI), Mayo score and the life quality index (LQI or IBDQ-D). One skilled in the art will appreciate that a such indices are common diagnostics used to evaluate the progression and/or efficacy of treatment of ulcerative colitis. It will be appreciated, however, that other available diagnostic can also be used to assess the efficacy of the methods described herein.

In some embodiments of the methods described herein, administration of phosphatidylcholine to an individual having steroid-dependent or steroid-refractory ulcerative colitis results in a endoscopic activity index (EAI) improvement of at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or 100%.

In some embodiments of the methods described herein, administration of phosphatidylcholine to an individual having steroid-dependent or steroid-refractory ulcerative colitis results in a histology index reduction of at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or 100%.

In some embodiments of the methods described herein, administration of phosphatidylcholine to an individual having steroid-dependent or steroid-refractory ulcerative colitis results in a clinical activity index improvement of at least of at least 5%, at least 10%, at least 15%, at least 20%, at least 25%, at least 30%. at least 35%, at least 40%, at least 45%, at least 50%, at least 55%, at least 60%, at least 65%, at least 70%, at least 75%, at least 80%, at least 85%, at least 90%, at least 95%, or 100%.

In some embodiments of the methods described herein, administration of phosphatidylcholine to an individual having steroid-dependent or steroid-refractory ulcerative colitis results in a quality of life index improvement of at least of at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or 100%.

Having generally described embodiments of the present invention, a further understanding can be obtained by reference to certain specific examples which are provided herein for purposes of illustration only.

### EXAMPLE 1

A double-blind, randomized study was conducted at Heidelberg University Hospital. A sample size of 60 patients was chosen in accordance with the procedure set out in Stremmel W, Merle U, Zahn A, Autshbach F, Hinz U, Ehehalt R., Retarded release phosphatidylcholine benefits patients with chronic active ulcerative colitis. Gut, 2005. 54, 966-71. One hundred forty four patients applied for the study, 131 of those had a confirmed ulcerative colitis and 84 a chronic active course. Sixty patients fulfilled the inclusion criteria of long-term steroid therapy with a continuous inflammatory activity over at least 4 months (mean 2.5 years, Table 1) and a clinical activity index (CAI) and endoscopic activity index (EAI) each ≥ 5 (steroid refractory course). The study protocol was approved by the institutional ethics committee according to the declaration of Helsinki and registered at ClinicalTrials.gov (No. NCT00259545). All patients gave informed written consent prior to their inclusion and could withdraw from study at any time.

**Table 1: Patients' Characteristics at Entry to the Study**

| **Treatment Group** | **PC group** | **placebo group** | **Total** | **p-value** |
|---|---|---|---|---|
| | (n=30) | (n=30) | (n=60) | |
| **Sex** | | | | 1.00 |
| male | 18 | 17 | 35 | |
| female | 12 | 13 | 25 | |
| | | | | |
| **Age (Mean)** | 37.7 (SD 10.4) | 35.4(SD 13.2) | 36.6 (SD11.9) | 0.28 |
| **extension of disease** | | | | 1.00 |
| pancolitis | 14 (46.7%) | 13 (43.3%) | 27 (45.0%) | |
| to right flexure | 2 (6.7%) | 2 (6.7%) | 4 (6.7%) | |
| to left flexure | 13 (43.3%) | 12 (40.0%) | 25 (41.7%) | |
| to SIGMA | 1 (3.3%) | 3 (10%) | 4 (6.7%) | |
| **mean prednisone equivalent** | 18.8mg (SD 12.3) | 14.6mg (SD 8.7) | 16.7mg (SD 10.8) | 0.15 |
| **duration of steroid intake (yrs.)** | 2.3 (SD 12.3) | 2.2 (SD 2.0) | 2.2 (SD 2.4) | 0.67 |
| **duration of active course (yrs.)** | 3.1 (SD 3.5) | 1.9 (SD 1.8) | 2.5 (SD 2.8) | 0.49 |
| **duration of disease (yrs.)** | 10.2 (SD 6.9) | 6.9 (SD 5.9) | 8.6 (SD 6.6) | 0.06 |
| **concomitant medication** | 24 | 21 | | 0.55 |
| 5-ASA preparations | 21 | 19 | | |
| E. coli Nissle 1917 | 4 | 3 | | |
| **CAI (mean)** | 8.6 (SD 2.7) | 7.0 (SD 1.7) | 7.8 (SD 2.4) | 0.04 |
| **EAI (mean)** | 7.4 (SD 1.3) | 7.1 (SD 1.5) | 7.3 (SD 1.4) | 0.45 |
| **histology - index (mean)** | 2.7 (SD 0.9) | 3.1 (SD 0.9) | 2.9 (SD 0.9) | 0.13 |
| **IBDQ-D (mean)** | 110.7 (SD 27.4) | 124.3 (SD 26.2) | 117.5 (SD 27.4) | 0.05 |

A clinical evaluation with colonoscopy and serial biopsies was performed after a screening phase of at least ten days and after the 12-week study period. The basic medication at study entry of 5-aminosalicylates and/or E. coli Nissle in 75% of the patients was continued during the study and changes of basic medication were strongly discouraged. Fifty-three patients did not take immunosuppressives; eighteen due to a lack of efficacy (30.0%), twelve had adverse events from azathioprine/6-mercaptopurine (20.0%) and twenty-three refused these drugs due to possible complications (38.0%). Seven patients on azathioprine/6-mercaptopurine had been on the medication for at least six months and had to be maintained on the same dosage during the study. Patients on cyclosporine, tacrolimus or methotrexate were excluded from the study. Rectal applications were not accepted during the study to avoid interference with the endoscopic and histologic indices. Patients were asked to fill out a daily diary sheet which included number of bowel movements, quantity of blood in stool, wellbeing, abdominal pain, extraintestinal manifestations, fever and actual medication.

After receiving the study medication for two weeks, all patients were instructed to reduce their steroid dose if possible. Weekly telephone interviews with each patient were performed. Depending on the starting dose, steroids were reduced weekly by 10 mg prednisone equivalent reduction steps. When a 10 mg dosage was reached, steroids were reduced in weekly steps to 7.5, 5.0, 2.5 and 0 mg. If the symptoms deteriorated by 25% (concerning stool frequency, indices for blood in stool, abdominal pain and general wellbeing) the steroid reduction was paused or in case of acute inflammatory episodes a rescue therapy with 50 mg prednisone for three days was initiated, followed by the next higher steroid dose of the reduction regimen. Every change in steroid dosage was decided on by a panel of three specialists.

The study medication was prepared by Allphamed-NextPharma, Gottingen in the manner detailed in Stremmel W, Merle U, Zahn A, Autshbach F, Hinz U, Ehehalt R., Retarded release phosphatidylcholine benefits patients with chronic active ulcerative colitis. Gut, 2005. 54, 966-71. Both study medications (Eudragit S100 encapsulated PC and cellulose as placebo) were manufactured as indistinguishable granular preparations and packaged in numbered boxes using random permuted blocks of six patients. The randomization list was given to an independent study control (KKS Heidelberg) which forwarded the list after data base closure to the statistician. The study medication was administered orally QTD (0.5 g of PC or placebo per dose) after meals, resulting in 2 g of PC or cellulose per day. The 2 g of cellulose daily in the placebo group was not considered to be relevant as normal food contains a much higher dose with approximately 16 g (SD 7) of cellulose per day. Compliance was controlled by telephone interviews, return of the empty medication boxes and daily diary sheets.

Efficacy was determined by CAI and EAI as well as by a life quality index (IBDQ-D). The histology of rectal biopsies from 10 and 20 cm ab ano was evaluated by an independent pathologist blinded to clinical information using a modified scale of Truelove and Richards' index parameters as set forth in Stremmel W, Merle U, Zahn A, Autshbach F, Hinz U, Ehehalt R., Retarded release phosphatidylcholine benefits patients with chronic active ulcerative colitis. Gut, 2005. 54, 966-71.

The primary endpoint was defined as steroid withdrawal at the end of the study combined with either an accompanying achievement of a clinically inactive state (CAI ≤ 3) or a ≥ 50% CAI reduction compared to baseline (complete response).

Secondary endpoints included: [1] partial response defined as a complete steroid withdrawal and a ≤ 50% CAI improvement, but no CAI deterioration ≥20%; [2] Improvement of CAI, EAI, histology and life quality indices irrespective of steroid withdrawal. The number of patients who responded was compared between both treatment groups. For the CAI and EAI-categories, patients were divided between those who reached a clinically inactive state (index ≤ 3) and those who achieved ≥ E 50% improvement. For the histology and the IBDQ-D indices, a ≥ 25% improvement of the score was taken as a discriminate value. Additionally, the CAI, EAI, histologic and IBDQ-D indices were evaluated by calculating absolute and relative changes from baseline to the end of study.

All adverse events were recorded in a protocol. White blood cell count, hemoglobin, platelets, C-reactive protein, creatinine, urea, alanine aminotransferase, aspartate aminotransferase, gamma glutamyl transferase and total bilirubin were determined at entry and end of study as laboratory safety parameters. Since there were no relevant differences of these safety parameters observed between placebo and PC during the study, detailed data are not provided here.

All statistical analyses were performed using SAS software (Release 9.1.3, SAS Institute, Inc., Cary, NC). The analyses were performed with an intent to treat (ITT) approach. Thus, all patients treated and having at least one efficacy parameter postbaseline were included in the analysis of efficacy. An additional per protocol analysis was performed which confirmed the ITT analyses. The data were equivalent and therefore not provided here.

Comparisons of primary and secondary variables between the two treatment groups as well as the comparability of treatment groups at study entry for categorical data were performed using Fisher's exact test. Mann-Whitney U tests were performed to detect differences between PC and placebo for continuous variables at study entry as well as absolute and relative changes from baseline to end of study. Furthermore, absolute and relative changes from baseline to end of study were investigated within both treatment groups using Wileoxon's signed rank test. Continuous variables were expressed as mean values with standard deviation (SD). The distribution of absolute changes in CAI, EAI, histology score and LQI from baseline to end of study was presented by box-and-whisker plots (Figures 1A-D).

The demographic data in the PC and placebo group were comparable with a trend towards higher disease activity in the PC group (Table 1). This trend, however, did not interfere with the achievement of primary or secondary variables as demonstrated by the dichotomous endpoint analysis (Tables 2 and 3).

**Table 2: Response Rates of Primary Endpoint Variables**

| Treatment-Group | PC Group | placebo-group | p-value |
|---|---|---|---|
| **complete response** | | | |
| steroid withdrawal and low clinical activity (CAI≤3) | 12 | 3 | 0.02 |
| steroid withdrawal and ≥ 50% reduction of CAI (with CAI<3) | 3 | 0 | |
| **total** | **15** | **3** | 0.02 |
| | | | |

| **partial response** | | | |
|---|---|---|---|
| steroid withdrawal and < 50% reduction of CAI | 9 | 0 | <0.001 |
| | | | |

| **non-response** | | | |
|---|---|---|---|
| ≥50% steroid reduction | 1 | 2 | |
| , 50% steroid reduction | 5 | 25 | |
| **total** | **6** | **27** | <0.001 |

**Table 3: Response Rates of Secondary Endpoint Variables**

| | **PC group (n = 30)** | **placebo (n = 30)** | **P-value** |
|---|---|---|---|
| **CAI ≤ 3 at end of study** | | | 0.03 |
| achieved | 14 | 5 | |
| not achieved | 16 | 25 | |
| **Δ CAI ( ≥ 50%)** | | | 0.03 |
| achieved | 17 | 5 | |
| not achieved | 13 | 25 | |
| **EAI ≤ 3 at end of study** | | | <0.001 |
| achieved | 18 | 1 | |
| not achieved | 12 | 29 | |
| **Δ EAI ( ≥ 50%)** | | | <0.001 |
| achieved | 21 | 1 | |
| not achieved | 9 | 29 | |
| **A histology score ( ≥ 25%)** | | | 0.29 |
| achieved | 15 | 10 | |
| not achieved | 15 | 20 | |
| **Δ IBDQ-D ( ≥ 25%)** | | | 0.001 |
| achieved | 19 | 6 | |
| not achieved | 11 | 24 | |

| | | | |
|---|---|---|---|
| Note: Two dropouts in the PC group with missing data were treated as 'not achieved.' | | | |

Steroid withdrawal and a concomitant achievement of a clinically inactive state (CAI ≤ 3) were recorded in 12 of the patients (40%) in the PC group, whereas only in three patients (10%) under placebo (p=0.02). Additionally, three patients in the PC group were fully withdrawn from steroids and improved their CAI by ≥50%. Thus, the primary endpoint of steroid withdrawal and CAI ≤ 3 or CAI improvement ≥ 50% (complete response) was achieved in 50% of patients in the PC group and in 10% of patients in the placebo group (p=0.002) (table 2). There were an additional nine patients (30%) under PC (but none under placebo) who withdrew from steroids but whose CAI neither improved by ≥ 50% nor deteriorated by ≥ 20% (partial response) (p≤0.001). Among the 15 complete responders in the PC group, 13 showed a ≥50% improvement of their EAI (EAI ≥ 3 in 12 patients) and of those 12 with low endoscopic activity, seven also achieved a ≥ 25 % improvement of the histology index. Six non-responders were registered in the PC group, whereas the placebo group reported 27 non-responders (p≤0.001). This includes two patients without end of study measurements in the PC group and one screening failure under placebo (ITT).

Two cut off values were chosen: [1] the absolute threshold value of CAI ≤ 3 defining inactive disease (16) and [2] the relative improvement in CAI ≥ 50% (Table 3). Inactive disease was observed in 14 PC-treated patients (46.7%) compared to 5 patients (16.7%) in the placebo control (p=0.03). Improvement in CAI ≥ 50% was recorded in 17 PC patients (56.7%), but only in 5 (16.7%) placebo patients (p 0.003). With regard to the magnitude of change, the mean CAI in the PC group decreased from 8.6 [SD 2.7] to 4.1 [SD 3.7] (p≤0.001) versus a drop from 7.0 [SD 1.7] to 6.4 [SD 5.0] in the placebo group (p=0.08) (Figure 1A). This corresponds to a relative improvement of 52.0% under PC compared to 8.6% under placebo (p≤0.001).

In parallel to the CAI there was a ≥ 50% improvement of EAI in 21 patients of the PC group (18 with a EAI ≤ 3) compared to one patient in the placebo group (p≤0.001) (table 3). The mean reduction in the index score decreased from 7.4 [SD 1.3] to 3.7 [SD 1.9] (p≤0.001) in PC patients but remained almost unchanged in placebo patients (7.1 [SD 1.5] to 7.2 [SD 1.7] (p=0.15)) (Figure 1B). This corresponds to a relative improvement of 50.6% under PC compared to a 2.5% deterioration under placebo (p≤0.001).

A ≥ 25% reduction of the histology index was observed in 15 patients (50.0%) in the PC group compared to 10 (33%) in the placebo group (p=0.29) (table 3). The mean value of the index fell in PC patients from 2.7 [SD 0.9] to 2.2 [SD 1.1] (p=0.004) whereas placebo patients maintained a nearly unchanged mean histology score of 3.1 [SD 0.9] and 2.9 [SD 1.1] (p=0.33) (Figure 1 C). This corresponds to a relative improvement of 18.6% under PC compared to 0.7% under placebo (p=0.04).

All subjective quality of life parameters, including bowel symptoms, systemic symptoms, and emotional and social functions were recorded by the employed IBDQ-D. A score improvement of the ≥ 25% cut-off criterion was achieved in 19 patients (63%) in the PC group and six (20%) in the placebo group (p=0.001). The mean score increased from 110.7 [SD 27.4] to 158.3 [SD 37.3] (p<0.001) under PC and from 124.3 [SD 26.2] to 136.7 [SD 35.6] (p=0.09) under placebo (Figure 1D). This corresponds to a relative improvement of 49.5% under PC compared to 12.6% under placebo (p≤0.001).

Two patients in the PC group withdrew from the study, one due to an acute inflammatory episode and one due to non-compliance (refusal of final examination). In the placebo group, one patient withdrew early from the study due to clinical deterioration. One patient on placebo was considered screening failure, as methotrexate treatment was begun within the screening phase.

During the study the most common adverse event was bloating which was observed in 11 PC and 6 placebo patients (p=0.30), (Table 4). One patient in the PC group experienced a herniated vertebral disk which was not considered as study-related. The other registered adverse events were randomly distributed among both treatment groups (p=0.86).

**Table 4: Adverse Events in the PC and Placebo Groups**

| Adverse Event | PC. | I° | II° | III° | Placebo | I° | II° | III° | P-value |
|---|---|---|---|---|---|---|---|---|---|
| anal incontinence | | | | | 1 | 1 | | | |
| bloating | 11 | 11 | | | 6 | 5 | 1 | | 0.30 |
| diarrhea | 1 | | 1 | | 1 | | 1 | | |
| diffuse alopecia | 1 | 1 | | | 1 | | 1 | | |
| diminished appetite | 1 | 1 | | | | | | | 1.00 |
| dysphagia | | | | | 1 | 1 | | | |
| edema | 1 | 1 | | | | | | | 1.00 |
| fatigue/lassitude | 1 | 1 | | | 1 | 1 | | | |
| gastroenteritis | | | | | 1 | 1 | | | |
| headache | 2 | 2 | | | | | | | 0.49 |
| herniated vertebral disk | 1 | | | 1 | | | | | 1.00 |
| hot flash | 2 | 2 | | | 1 | 1 | | | 1.00 |
| hyperhidrosis | 3 | 3 | | | 2 | 2 | | | 1.00 |
| increased appetite | 2 | 2 | | | 1 | 1 | | | 1.00 |
| jaundice | 1 | 1 | | | 1 | 1 | | | |
| mucous stools | | | | | 2 | 2 | | | |
| muscular cramps | 1 | 1 | | | | | | | |
| nausea | 1 | 1 | | | 2 | 2 | | | |
| paresthesia | | | | | 1 | 1 | | | |
| rash | 1 | 1 | | | 2 | 1 | 1 | | |
| | | | | | | | | | |
| severe exacerbation of colitis | 1 | | | 1 | 1 | | | 1 | |
| sleep disorder | 1 | 1 | | | | | | | 1.00 |
| vertigo | 1 | 1 | | | 1 | 1 | | | |
| weight loss | 1 | 1 | | | 1 | 1 | | | |
| xerodermia | 1 | | 1 | | 3 | 3 | | | |
| xerostomia | | | | | 2 | 2 | | | |
| total | 34 | 30 | 2 | 2 | 31 | 26 | 4 | 1 | 0.86 |

The methods and compositions described herein are presently representative of preferred embodiments and are exemplary.

## Claims

1. Phosphatidylcholine for use in a method of ameliorating at least one symptom of ulcerative colitis, said method comprising:
identifying an individual having steroid-dependent ulcerative colitis; and
administering to said individual a therapeutically effective amount of phosphatidylcholine, thereby ameliorating at least one symptom of said ulcerative colitis.

2. Phosphatidylcholine for use in the method of claim 1, wherein said phosphatidylcholine is administered in a delayed release oral formulation,
wherein, preferably, the delayed release is pH-dependent,
wherein, preferably, the pH-dependent delayed release formulation comprises a gastric acid resistant substance,
wherein the gastric acid resistant substance is preferably selected from the group consisting of film covershields and carrier matrices, or wherein the gastric acid resistant substance comprises preferably acrylpolymers, wherein said acrylpolymer is preferably Eudragit, more preferably Eudragit S100, or wherein said acrylpolymer is Eudragit L30,
wherein, preferably, the pH-dependent delayed release oral formulation comprises about 500 mg phosphatidylcholine.

3. Phosphatidylcholine for use in the method of claim 1, wherein said therapeutically effective amount of phosphatidylcholine ranges from about 100 mg to about 8 g per day,
or wherein said therapeutically effective amount of phosphatidylcholine ranges from about 500 mg to about 4 g per day,
or wherein said therapeutically effective amount of phosphatidylcholine is about 2 g per day.

4. Phosphatidylcholine for use in the method of claim 1, wherein said phosphatidylcholine is administered near meal time.
or wherein said phosphatidylcholine is administered from about 1 to about 8 times per day,
or wherein said phosphatidylcholine is administered about 4 times per day,
or wherein the phosphatidylcholine is administered for at least 1 week,
or wherein the phosphatidylcholine is administered for at least 2 weeks,
or wherein the phosphatidylcholine is administered throughout the life of the individual.

5. Phosphatidylcholine for use in the method of claim 1, wherein administration results in a quality of life index score improvement of at least 25%,
or wherein administration results in a histology index reduction of at least 25%,
or wherein administration results in an endoscopic activity index improvement of at least 50%,
or wherein administration results in a clinical activity index improvement of at least 50%,
or wherein administration results in cessation of clinically active ulcerative colitis.

6. Phosphatidylcholine for use in the method of claim 1, wherein said steroid-dependence is ameliorated by at least about a 30% dose reduction of steroids administered to said individual as treatment for ulcerative colitis,
or wherein said dose reduction is at least about 50%,
or wherein said dose reduction is at least about 75%,
or wherein said dose reduction is 100%.

7. Phosphatidylcholine for use in a method of ameliorating at least one symptom of ulcerative colitis, said method comprising:
identifying an individual having steroid-dependent ulcerative colitis that has become steroid-refractory ulcerative colitis; and
administering to said individual a therapeutically effective amount of phosphatidylcholine, thereby ameliorating at least one symptom of said ulcerative colitis.

8. Phosphatidylcholine for use in the method of claim 7, wherein said phosphatidylcholine is administered in a delayed release oral formulation,
wherein, preferably, the delayed release is pH-dependent,
wherein, preferably, the pH-dependent delayed release formulation comprises a gastric acid resistant substance,
wherein the gastric acid resistant substance is preferably selected from the group consisting of film covershields and carrier matrices, or wherein the gastric acid resistant substance comprises preferably acrylpolymers, wherein said acrylpolymer is preferably Eudragit, more preferably Eudragit S 100 or Eudragit L30,
wherein, preferably, the pH-dependent delayed release oral formulation comprises about 500 mg phosphatidylcholine.

9. Phosphatidylcholine for use in the method of claim 7, wherein said therapeutically effective amount of phosphatidylcholine ranges from about 100 mg to about 8 g per day,
or wherein said therapeutically effective amount of phosphatidylcholine ranges from about 500 mg to about 4 g per day,
or wherein said therapeutically effective amount of phosphatidylcholine is about 2 g per day.

10. Phosphatidylcholine for use in the method of claim 7, wherein said phosphatidylcholine is administered near meal time,
or wherein said phosphatidylcholine is administered from about 1 to about 8 times per day, or wherein said phosphatidylcholine is administered about 4 times per day,
or wherein the phosphatidylcholine is administered for at least 1 week,
or wherein the phosphatidylcholine is administered for at least 2 weeks,
or wherein the phosphatidylcholine is administered throughout the life of the individual.

11. Phosphatidylcholine for use in the method of claim 7, wherein administration results in a quality of life index score improvement of at least 25%,
or wherein administration results in a histology index reduction of at least 25%,
or wherein administration results in an endoscopic activity index improvement of at least 50%,
or wherein administration results in a clinical activity index improvement of at least 50%,
or wherein administration results in cessation of clinically active ulcerative colitis.

12. Phosphatidylcholine for use in a method of ameliorating at least one symptom of ulcerative colitis comprising:
identifying an individual having steroid-refractory ulcerative colitis; and
administering to said individual a therapeutically effective amount of phosphatidylcholine, thereby ameliorating at least one symptom of said ulcerative colitis.

13. Phosphatidylcholine for use in the method of claim 12, wherein said phosphatidylcholine is administered in a delayed release oral formulation,
wherein, preferably, the delayed release is pH-dependent,
wherein, preferably, the pH-dependent delayed release formulation comprises a gastric acid resistant substance,
wherein the gastric acid resistant substance is preferably selected from the group consisting of film covershields and carrier matrices, or wherein the gastric acid resistant substance comprises preferably acrylpolymers, wherein said acrylpolymer is preferably Eudragit, more preferably Eudragit S100 or Eudragit L30,
wherein, preferably, the pH-dependent delayed release oral formulation comprises about 500 mg phosphatidylcholine.

14. Phosphatidylcholine for use in the method of claim 12, wherein said therapeutically effective amount of phosphatidylcholine ranges from about 100 mg to about 8 g per day,
or wherein said therapeutically effective amount of phosphatidylcholine ranges from about 500 mg to about 4 g per day,
or wherein said therapeutically effective amount of phosphatidylcholine is about 2 g per day.

15. Phosphatidylcholine for use in the method of claim 12, wherein said phosphatidylcholine is administered near meal time,
or wherein said phosphatidylcholine is administered from about 1 to about 8 times per day,
or wherein said phosphatidylcholine is administered about 4 times per day,
or wherein the phosphatidylcholine is administered for at least 1 week,
or wherein the phosphatidylcholine is administered for at least 2 weeks,
or wherein the phosphatidylcholine is administered throughout the life of the individual.

16. Phosphatidylcholine for use in the method of claim 12, wherein administration results in a quality of life index score improvement of at least 25%,
or wherein administration results in a histology index reduction of at least 25%,
or wherein administration results in an endoscopic activity index improvement of at least 50%,
or wherein administration results in a clinical activity index improvement of at least 50%,
or wherein administration results in cessation of clinically active ulcerative colitis.

## Patentansprüche

1. Phosphatidylcholin für die Anwendung in einem Verfahren zur Linderung von maindestens einem Symptom der Colitis ulcerosa, wobei dieses Verfahren umfasst:
Identifizieren einer Person mit Steroid-abhängiger Colitis ulcerosa; und
Verabreichen einer therapeutisch wirksamen Menge von Phosphatidylcholin an diese Person, um mindestens ein Symptom der Colitis ulcerosa zu lindern.

2. Phosphatidylcholin für die Anwendung in dem Verfahren gemäß Anspruch 1, wobei Phosphatidylcholin in oraler Darreichungsform mit verzögerter Freisetzung verabreicht wird, wobei die verzögerte Freisetzung vorzugsweise pH-abhängig ist,
wobei die Darreichungsform mit pH-abhängiger verzögerter Freisetzung vorzugsweise eine magensäureresistente Substanz umfasst,
wobei die magensäureresistente Substanz vorzugsweise ausgewählt ist aus der Gruppe bestehend aus umhüllendem Schutzfilm und Träger-Matrizen, oder wobei die magensäureresistente Substanz vorzugsweise Acrylpolymere umfasst, wobei das Acrylpolymer vorzugsweise Eudragit, bevorzugter Eudragit S 100 ist, oder wobei das Acrylpolymer Eudragit L30 ist, wobei die Darreichungsform mit pH-abhängiger verzögerter Freisetzung vorzugsweise ungefähr 500 mg Phosphatidylcholin enthält.

3. Phosphatidylcholin für die Anwendung in dem Verfahren gemäß Anspruch 1, wobei die therapeutisch wirksame Menge an Phosphatidylcholin im Bereich von ungefähr 100 mg bis ungefähr 8 g pro Tag liegt,
oder wobei die therapeutisch wirksame Menge an Phosphatidylcholin im Bereich von ungefähr 500 mg bis ungefähr 4 g pro Tag liegt,
oder wobei die therapeutisch wirksame Menge an Phosphatidylcholin ungefähr 2 g pro Tag ist.

4. Phosphatidylcholin für die Anwendung in dem Verfahren gemäß Anspruch 1, wobei Phosphatidylcholin ungefähr zur Essenszeit verabreicht wird,
oder wobei das Phosphatidylcholin von ungefähr 1- bis ungefähr 8-mal am Tag verabreicht wird,
oder wobei das Phosphatidylcholin ungefähr 4-mal am Tag verabreicht wird,
oder wobei das Phosphatidylcholin mindestens eine Woche verabreicht wird,
oder wobei das Phosphatidylcholin für mindestens zwei Wochen verabreicht wird,
oder wobei das Phosphatidylcholin während des gesamten Lebens der Person verabreicht wird.

5. Phosphatidylcholin für die Anwendung in dem Verfahren gemäß Anspruch 1, wobei die Verabreichung zu einer Erhöhung des Lebensqualitätsindexes von mindestens 25 % führt, oder wobei die Verabreichung zu einer Verringerung des Histologie-Indexes von mindestens 25 % führt,
oder wobei die Verabreichung zu einer Verbesserung des endoskopischen Aktivitätsindexes von mindestens 50 % führt,
oder wobei die Verabreichung zu einer Verbesserung des klinischen Aktivitäts-Indexes von mindestens 50 % führt,
oder wobei die Verabreichung zu einem Wegfall der klinisch aktiven Colitis ulcerosa führt.

6. Phosphatidylcholin für die Anwendung in dem Verfahren gemäß Anspruch 1, wobei die Steroid-Abhängigkeit gelindert ist durch eine Verringerung der zur Behandlung der Colitis ulcerosa an die Person verabreichten Steroiddosis um mindestens ungefähr 30 % ist,
oder wobei die Verringerung der Dosis mindestens ungefähr 50 % ist,
oder wobei die Verringerung der Dosis mindestens ungefähr 75 % ist,
oder wobei die Verringerung der Dosis 100 % verringert ist.

7. Phosphatidylcholin für die Anwendung in einem Verfahren zur Linderung von mindestens einem Symptom der Colitis ulcerosa, wobei dieses Verfahren umfasst:
Identifizieren einer Person mit Steroid-abhängiger Colitis ulcerosa, welche zu einer Steroid-refraktären Colitis ulcerosa geworden ist; und
Verabreichen einer therapeutisch wirksamen Menge von Phosphatidylcholin an diese Person, um mindestens ein Symptom der Colitis ulcerosa zu lindern.

8. Phosphatidylcholin für die Anwendung in dem Verfahren gemäß Anspruch 7, wobei Phosphatidylcholin in oraler Darreichungsform mit verzögerter Freisetzung verabreicht wird, wobei die verzögerte Freisetzung vorzugsweise pH-abhängig ist,
wobei die Darreichungsform mit pH-abhängiger verzögerter Freisetzung vorzugsweise eine magensäureresistente Substanz umfasst,
wobei die magensäureresistente Substanz vorzugsweise ausgewählt ist aus der Gruppe bestehend aus umhüllendem Schutzfilm und Träger-Matrizen, oder wobei die magensäureresistente Substanz vorzugsweise Acrylpolymere umfasst, wobei das Acrylpolymer vorzugsweise Eudragit, bevorzugter Eudragit S100 ist, oder wobei das Acrylpolymer Eudragit L30 ist, wobei die Darreichungsform mit pH-abhängiger verzögerter Freisetzung vorzugsweise ungefähr 500 mg Phosphatidylcholin enthält.

9. Phosphatidylcholin für die Anwendung in dem Verfahren gemäß Anspruch 7, wobei die therapeutisch wirksame Menge an Phosphatidylcholin im Bereich von ungefähr 100 mg bis ungefähr 8 g pro Tag liegt,
oder wobei die therapeutisch wirksame Menge an Phosphatidylcholin im Bereich von ungefähr 500 mg bis ungefähr 4 g pro Tag liegt,
oder wobei die therapeutisch wirksame Menge an Phosphatidylcholin ungefähr 2 g pro Tag ist.

10. Phosphatidylcholin für die Anwendung in dem Verfahren gemäß Anspruch 7, wobei Phosphatidylcholin ungefähr zur Essenszeit verabreicht wird,
oder wobei das Phosphatidylcholin von ungefähr 1- bis ungefähr 8-mal am Tag verabreicht wird,
oder wobei das Phosphatidylcholin ungefähr 4-mal am Tag verabreicht wird,
oder wobei das Phosphatidylcholin mindestens eine Woche verabreicht wird,
oder wobei das Phosphatidylcholin für mindestens zwei Wochen verabreicht wird,
oder wobei das Phosphatidylcholin während des gesamten Lebens der Person verabreicht wird.

11. Phosphatidylcholin für die Anwendung in dem Verfahren gemäß Anspruch 7, wobei die Verabreichung zu einer Erhöhung des Lebensqualitätsindexes von mindestens 25 % führt, oder wobei die Verabreichung zu einer Verringerung des Histologie-Indexes von mindestens 25 % führt,
oder wobei die Verabreichung zu einer Verbesserung des endoskopischen Aktivitätsindexes von mindestens 50 % führt,
oder wobei die Verabreichung zu einer Verbesserung des klinischen Aktivitäts-Indexes von mindestens 50 % führt,
oder wobei die Verabreichung zu einem Wegfall der klinisch aktiven Colitis ulcerosa führt.

12. Phosphatidylcholin für die Anwendung in einem Verfahren zur Linderung von mindestens einem Symptom der Colitis ulcerosa umfassend:
Identifizieren einer Person mit Steorid-refraktärer Colitis ulcerosa; und
Verabreichen einer therapeutisch wirksamen Menge von Phosphatidylcholin an diese Person, um mindestens ein Symptom der Colitis ulcerosa zu lindern.

13. Phosphatidylcholin für die Anwendung in dem Verfahren gemäß Anspruch 12, wobei Phosphatidylcholin in oraler Darreichungsform mit verzögerter Freisetzung verabreicht wird, wobei die verzögerte Freisetzung vorzugsweise pH-abhängig ist,
wobei die Darreichungsform mit pH-abhängiger verzögerter Freisetzung vorzugsweise eine magensäureresistente Substanz umfasst,
wobei die magensäureresistente Substanz vorzugsweise ausgewählt ist aus der Gruppe bestehend aus umhüllendem Schutzfilm und Träger-Matrizen, oder wobei die magensäureresistente Substanz vorzugsweise Acrylpolymere umfässt, wobei das Acrylpolymer vorzugsweise Eudragit, bevorzugter Eudragit S100 ist, oder wobei das Acrylpolymer Eudragit L30 ist, wobei die Darreichungsform mit pH-abhängiger verzögerter Freisetzung vorzugsweise ungefähr 500 mg Phosphatidylcholin enthält.

14. Phosphatidylcholin für die Anwendung in dem Verfahren gemäß Anspruch 12, wobei die therapeutisch wirksame Menge an Phosphatidylcholin im Bereich von ungefähr 100 mg bis ungefähr 8 g pro Tag liegt,
oder wobei die therapeutisch wirksame Menge an Phosphatidylcholin im Bereich von ungefähr 500 mg bis ungefähr 4 g pro Tag liegt,
oder wobei die therapeutisch wirksame Menge an Phosphatidylcholin ungefähr 2 g pro Tag ist.

15. Phosphatidylcholin für die Anwendung in dem Verfahren gemäß Anspruch 12, wobei Phosphatidylcholin ungefähr zur Essenszeit verabreicht wird,
oder wobei das Phosphatidylcholin von ungefähr 1- bis ungefähr 8-mal am Tag verabreicht wird,
oder wobei das Phosphatidylcholin ungefähr 4-mal am Tag verabreicht wird,
oder wobei das Phosphatidylcholin mindestens eine Woche verabreicht wird,
oder wobei das Phosphatidylcholin für mindestens zwei Wochen verabreicht wird,
oder wobei das Phosphatidylcholin während des gesamten Lebens der Person verabreicht wird.

16. Phosphatidylcholin für die Anwendung in dem Verfahren gemäß Anspruch 12, wobei die Verabreichung zu einer Erhöhung des Lebensqualitätsindexes von mindestens 25 % führt, oder wobei die Verabreichung zu einer Verringerung des Histologie-Indexes von mindestens 25 % führt,
oder wobei die Verabreichung zu einer Verbesserung des endoskopischen Aktivitätsindexes von mindestens 50 % führt,
oder wobei die Verabreichung zu einer Verbesserung des klinischen Aktivitäts-Indexes von mindestens 50 % führt,
oder wobei die Verabreichung zu einem Wegfall der klinisch aktiven Colitis ulcerosa führt.

## Revendications

1. Phosphatidylcholine pour une utilisation dans un procédé d'amélioration d'au moins un symptôme de colite ulcéreuse, ledit procédé comprenant :
- l'identification d'un individu présentant une colite ulcéreuse stéroïdodépendante, et
- l'administration audit individu d'une quantité efficace du point de vue thérapeutique de phosphatidylcholine, de façon à améliorer au moins un symptôme de ladite colite ulcéreuse.

2. Phosphatidylcholine pour une utilisation dans le procédé selon la revendication 1, dans laquelle ladite phosphatidylcholine est administrée dans une formulation orale à effet retard,
dans laquelle, de préférence, l'effet retard dépend du pH,
dans laquelle, de préférence, la formulation à effet retard pH-dépendante comprend une substance résistant à l'acide gastrique,
dans laquelle la substance résistant à l'acide gastrique est de préférence choisie dans le groupe consistant en des films covershields et des matrices support, ou dans laquelle la substance résistant à l'acide gastrique comprend de préférence des polymères acryliques, dans lesquels ledit polymère acrylique est de préférence l'Eudragit, plus particulièrement l'Eudragit S100 ou dans lesquels ledit polymère acrylique est l'Eudragit L30,
dans laquelle, de préférence, la formulation orale à effet retard pH-dépendante comprend environ 500 mg de phosphatidylcholine.

3. Phosphatidylcholine pour une utilisation dans le procédé selon la revendication 1, dans laquelle ladite quantité efficace du point de vue thérapeutique de phosphatidylcholine est va d'environ 100 mg à environ 8 g par jour,
ou dans laquelle ladite quantité efficace du point de vue thérapeutique de phosphatidylcholine est va d'environ 500 mg à environ 4 g par jour,
ou dans laquelle ladite quantité efficace du point de vue thérapeutique de phosphatidylcholine est d'environ 2 g par jour.

4. Phosphatidylcholine pour une utilisation dans le procédé selon la revendication 1, dans laquelle ladite phosphatidylcholine est administrée aux environs du moment du repas,
ou dans laquelle ladite phosphatidylcholine est administrée à raison d'environ 1 à environ 8 fois par jour,
ou dans laquelle ladite phosphatidylcholine est administrée à raison d'environ 4 fois par jour,
ou dans laquelle ladite phosphatidylcholine est administrée pendant au moins 1 semaine,
ou dans laquelle ladite phosphatidylcholine est administrée pendant au moins 2 semaines,
ou dans laquelle ladite phosphatidylcholine est administrée pendant toute la vie de l'individu.

5. Phosphatidylcholine pour une utilisation dans le procédé selon la revendication 1, dans laquelle l'administration conduit à une amélioration du score de l'indice de qualité de vie d'au moins 25%,
ou dans laquelle l'administration conduit à une réduction de l'indice d'histologie d'au moins 25%,
ou dans laquelle l'administration conduit à une amélioration de l'indice d'activité endoscopique d'au moins 50%,
ou dans laquelle l'administration conduit à une amélioration de l'indice d'activité clinique d'au moins 50%,
ou dans laquelle l'administration conduit à une cessation de la colite ulcéreuse cliniquement active.

6. Phosphatidylcholine pour une utilisation dans le procédé selon la revendication 1, dans laquelle la dépendance vis-à-vis des stéroïdes est améliorée par une réduction d'au moins environ 30% de la dose des stéroïdes administrée audit individu pour le traitement de la colite ulcéreuse,
ou dans laquelle ladite réduction de dose est au moins d'environ 50%,
ou dans laquelle ladite réduction de dose est au moins d'environ 75%,
ou dans laquelle ladite réduction de dose est de 100%.

7. Phosphatidylcholine pour une utilisation dans un procédé d'amélioration d'au moins un symptôme de colite ulcéreuse, ledit procédé comprenant :
- l'identification d'un individu présentant une colite ulcéreuse dépendante des stéroïdes, qui est devenue une colite ulcéreuse stéroïdo-résistante. et
- l'administration audit individu d'une quantité efficace du point de vue thérapeutique de phosphatidylcholine, de façon à améliorer au moins un symptôme de ladite colite ulcéreuse.

8. Phosphatidylcholine pour une utilisation dans le procédé selon la revendication 7, dans laquelle ladite phosphatidylcholine est administrée dans une formulation orale à effet retard,
dans laquelle, de préférence, l'effet retard dépend du pH,
dans laquelle, de préférence, la formulation à effet retard pH-dépendante comprend une substance résistant à l'acide gastrique,
dans laquelle la substance résistant à l'acide gastrique est de préférence choisie dans le groupe consistant en des films covershields et des matrices support, ou dans laquelle la substance résistant à l'acide gastrique comprend de préférence des polymères acryliques, dans lesquels ledit polymère acrylique est de préférence l'Eudragit, plus particulièrement l'Eudragit S100 ou dans lesquels ledit polymère acrylique est l'Eudragit L30,
dans laquelle, de préférence, la formulation orale à effet retard pH-dépendante comprend environ 500 mg de phosphatidylcholine.

9. Phosphatidylcholine destinée à être utilisée dans le procédé selon la revendication 7, dans laquelle ladite quantité efficace du point de vue thérapeutique de phosphatidylcholine est comprise entre environ 100 mg et environ 8 g par jour, ou dans laquelle ladite quantité efficace du point de vue thérapeutique de phosphatidylcholine est comprise entre environ 500 mg et environ 4 g par jour,
ou dans laquelle ladite quantité efficace du point de vue thérapeutique de phosphatidylcholine est d'environ 2 g par jour.

10. Phosphatidylcholine pour une utilisation dans le procédé selon la revendication 7, dans laquelle ladite phosphatidylcholine est administrée aux environs du moment du repas,
ou dans laquelle ladite phosphatidylcholine est administrée à raison d'environ 1 à environ 8 fois par jour,
ou dans laquelle ladite phosphatidylcholine est administrée à raison d'environ 4 fois par jour,
ou dans laquelle ladite phosphatidylcholine est administrée pendant au moins 1 semaine,
ou dans laquelle ladite phosphatidylcholine est administrée pendant au moins 2 semaines,
ou dans laquelle ladite phosphatidylcholine est administrée pendant toute la vie de l'individu.

11. Phosphatidylcholine pour une utilisation dans le procédé selon la revendication 7, dans laquelle l'administration conduit à une amélioration du score de l'indice de qualité de vie d'au moins 25%,
ou dans laquelle l'administration conduit à une réduction de l'indice d'histologie d'au moins 25%,
ou dans laquelle l'administration conduit à une amélioration de l'indice d'activité endoscopique d'au moins 50%,
ou dans laquelle l'administration conduit à une amélioration de l'indice d'activité clinique d'au moins 50%,
ou dans laquelle l'administration conduit à une cessation de la colite ulcéreuse cliniquement active.

12. Phosphatidylcholine pour une utilisation dans un procédé d'amélioration d'au moins un symptôme de colite ulcéreuse comprenant :
- l'identification d'un individu présentant une colite ulcéreuse stéroïdorésistante, et
- l'administration audit individu d'une quantité efficace du point de vue thérapeutique de phosphatidylcholine, de façon à améliorer au moins un symptôme de ladite colite ulcéreuse.

13. Phosphatidylcholine pour une utilisation dans le procédé selon la revendication 12, dans laquelle ladite phosphatidylcholine est administrée dans une formulation orale à effet retard,
dans laquelle, de préférence, l'effet retard dépend du pH,
dans laquelle, de préférence, la formulation à effet retard pH-dépendante comprend une substance résistant à l'acide gastrique,
dans laquelle la substance résistant à l'acide gastrique est de préférence choisie dans le groupe consistant en des films covershields et des matrices support, ou dans laquelle la substance résistant à l'acide gastrique comprend de préférence des polymères acryliques, dans lesquels ledit polymère acrylique est de préférence l'Eudragit, plus particulièrement l'Eudragit S100 ou dans lesquels ledit polymère acrylique est l'Eudragit L30,
dans laquelle, de préférence, la formulation orale à effet retard pH-dépendante comprend environ 500 mg de phosphatidylcholine.

14. Phosphatidylcholine pour une utilisation dans le procédé selon la revendication 12, dans laquelle ladite quantité efficace du point de vue thérapeutique de phosphatidylcholine va d'environ 100 mg à environ 8 g par jour,
ou dans laquelle ladite quantité efficace du point de vue thérapeutique de phosphatidylcholine va d'environ 500 mg à environ 4 g par jour,
ou dans laquelle ladite quantité efficace du point de vue thérapeutique de phosphatidylcholine est d'environ 2 g par jour.

15. Phosphatidylcholine pour une utilisation dans le procédé selon la revendication 12, dans laquelle ladite phosphatidylcholine est administrée aux environs du moment du repas,
ou dans laquelle ladite phosphatidylcholine est administrée à raison d'environ 1 à environ 8 fois par jour,
ou dans laquelle ladite phosphatidylcholine est administrée à raison d'environ 4 fois par jour,
ou dans laquelle ladite phosphatidylcholine est administrée pendant au moins 1 semaine,
ou dans laquelle ladite phosphatidylcholine est administrée pendant au moins 2 semaines,
ou dans laquelle ladite phosphatidylcholine est administrée pendant toute la vie de l'individu.

16. Phosphatidylcholine pour une utilisation dans le procédé selon la revendication 12, dans laquelle l'administration conduit à une amélioration du score de l'indice de qualité de vie d'au moins 25%,
ou dans laquelle l'administration conduit à une réduction de l'indice d'histologie d'au moins 25%,
ou dans laquelle l'administration conduit à une amélioration de l'indice d'activité endoscopique d'au moins 50%,
ou dans laquelle l'administration conduit à une amélioration de l'indice d'activité clinique d'au moins 50%,
ou dans laquelle l'administration conduit à une cessation de la colite ulcéreuse cliniquement active.
